# EUROPEAN PATENT SPECIFICATION

(11) **EP 2 569 002 B1**
(45) Date of publication and mention of the grant of the patent: **26.10.2016**
(21) Application number: 11718398.8
(22) Date of filing: 09.05.2011
(51) Int. Cl.: A61K 38/28

(54) **PROCESS FOR THE PREPARATION OF INSULIN-ZINC COMPLEXES**
VERFAHREN ZUR HERSTELLUNG VON INSULIN-ZINK KOMLEXEN
PROCÉDÉ DE PRÉPARATION DE COMPLEXES D'INSULINE ET DE ZINC

(30) Priority: 11.05.2010 US 333497 P; 10.05.2010 EP 10162368
(43) Date of publication of application: 20.03.2013
(73) Proprietor: Novo Nordisk A/S, 2880 Bagsværd (DK)
(72) Inventor: ANDRESEN, Lene, DK-2760 Måløv (DK); HANSEN, Rosa Rebecca Erritzøe, DK-2400 København (DK); JEPPESEN, Per, DK-2700 Brønshøj (DK)
(86) International application number: PCT/EP2011/057388
(87) International publication number: WO 2011/141407

(56) References cited:
- WO-A1-98/47529
- WO-A2-2007/074133
- DE-B- 1 212 679
- GB-A- 1 042 194
- US-A- 3 868 358
- US-A1- 2009 312 236

## Description

### FIELD OF THE INVENTION

The present invention relates to a process for producing a pharmaceutical formulation comprising insulin and zinc, the pharmaceutical formulation obtainable by the process and to the use of the formulation for the treatment of diabetes.

### BACKGROUND OF THE INVENTION

Insulin is a 51 amino acid peptide hormone produced in the islets of Langerhans in the pancreas. Its primary function, acting as a monomer, is to facilitate the transport of glucose molecules across the cell membranes of adipose and muscle tissue by binding to and activating a transmembrane receptor.

Formulations of insulin are usually prepared by dissolving insulin in a small volume of water under acidic conditions. Zinc is then added to the formulation followed by a neutralisation and addition of preservatives like phenol and m-cresol.

WO 2005/012347 discloses insulin derivatives having a negatively charged side chain.

WO 2007/074133 discloses soluble pharmaceutical formulations comprising acylated insulin and more than 4 zinc atoms per 6 molecules of acylated insulin.

The present invention overcomes the problems of the prior art.

### SUMMARY OF THE INVENTION

The present invention relates to a process for preparing a pharmaceutical formulation comprising an insulin derivative, wherein the process comprises dissolving an insulin derivative in water, optionally comprising pharmaceutically acceptable excipients, to form a solution of insulin derivative, adjusting the pH of the solution to a pH above 7.2, adding a zinc solution during a period longer than seven minutes while stirring continuously and adjusting the pH to the target pH of the formulation, and wherein the insulin derivative comprises and insulin molecule having a side chain attached to the ε-amino group of a Lys residue present in the B chain of the parent insulin, the side chain being of the general formula:

-W-X-Y-Z.

The invention further relates to a product obtainable by the process and to the use of the process for the manufacture of a medicament for the treatment of diabetes.

### DEFINITIONS

The term **"pharmaceutical formulation"** as used herein means a product comprising an active compound or a salt thereof together with pharmaceutical excipients such as buffer, preservative and tonicity modifier, said pharmaceutical formulation being useful for treating, preventing or reducing the severity of a disease or disorder by administration of said pharmaceutical formulation to a person. Thus a pharmaceutical formulation is also known in the art as a pharmaceutical composition.

By **"target pH of the formulation"** is meant the pH, which is the desired pH value in the final pharmaceutical formulation.

The term **"pharmaceutically acceptable"** as used herein means suited for normal pharmaceutical applications, i.e. giving rise to no adverse events in patients etc.

The term "**insulin derivative"** as used herein means a chemically modified parent insulin or an analogue thereof, wherein the modification(s) are in the form of attachment of amides, carbohydrates, alkyl groups, acyl groups, esters, PEGylations, and the like. One example is LysB29Nε-hexadecandioyl-γ-Glu desB30 human insulin.

The term **"human insulin"** as used herein means the human insulin hormone whose structure and properties are well-known. Human insulin has two polypeptide chains, named the A-chain and the B-chain. The A-chain is a 21 amino acid peptide and the B-chain is a 30 amino acid peptide, the two chains being connected by disulphide bridges: a first bridge between the cysteine in position 7 of the A-chain and the cysteine in position 7 of the B-chain, and a second bridge between the cysteine in position 20 of the A-chain and the cysteine in position 19 of the B-chain. A third bridge is present between the cysteines in position 6 and 11 of the A-chain.

In the human body, the hormone is synthesized as a single-chain precursor proinsulin (preproinsulin) consisting of a prepeptide of 24 amino acids followed by proinsulin containing 86 amino acids in the configuration: prepeptide-B-Arg Arg-C-Lys Arg-A, in which C is a connecting peptide of 31 amino acids. Arg-Arg and Lys-Arg are cleavage sites for cleavage of the connecting peptide from the A and B chains.

The term **"insulin peptide"** as used herein means a peptide which is either human insulin or an analog or a derivative thereof with insulin activity.

The term **"parent insulin"** as used herein is intended to mean an insulin before any modifications of the amino acid sequence have been applied thereto.

The term **"insulin analogue"** as used herein means a modified insulin wherein one or more amino acid residues of the insulin have been substituted by other amino acid residues and/or wherein one or more amino acid residues have been deleted from the insulin and/or wherein one or more amino acid residues have been added and/or inserted to the insulin.

In one embodiment an insulin analogue comprises less than 8 modifications (substitutions, deletions, additions (including insertions) and any combination thereof) relative to the parent insulin, alternatively less than 7 modifications relative to the parent insulin, alternatively less than 6 modifications relative to the parent insulin, alternatively less than 5 modifications relative to the parent insulin, alternatively less than 4 modifications relative to the parent insulin, alternatively less than 3 modifications relative to the parent insulin, alternatively less than 2 modifications relative to the parent insulin. One example of an insulin analogue is AspB28 human insulin.

Modifications in the insulin molecule are denoted stating the chain (A or B), the position, and the one or three letter code for the amino acid residue substituting the native amino acid residue.

By **"desB30"** or **"B(1-29)"** is meant a natural insulin B chain or an analogue thereof lacking the B30 amino acid and "A(1-21)" means the natural insulin A chain. Thus, e.g., A21Gly, B28Asp,desB30 human insulin is an analogue of human insulin where the amino acid in position 21 in the A chain is substituted with glycine, the amino acid in position 28 in the B chain is substituted with aspartic acid, and the amino acid in position 30 in the B chain is deleted.

Herein terms like **"A1", "A2"** and **"A3"** etc. indicates the amino acid in position 1, 2 and 3 etc., respectively, in the A chain of insulin (counted from the N-terminal end). Similarly, terms like B1, B2 and B3 etc. indicates the amino acid in position 1, 2 and 3 etc., respectively, in the B chain of insulin (counted from the N-terminal end). Using the one letter codes for amino acids, terms like A21A, A21G and A21Q designates that the amino acid in the A21 position is A, G and Q, respectively. Using the three letter codes for amino acids, the corresponding expressions are A21Ala, A21Gly and A21Gln, respectively.

Herein the terms "**A(0)**" or "**B(0)**" indicate the positions of the amino acids N-terminally to A1 or B1, respectively. The terms A(-1) or B(-1) indicate the positions of the first amino acids N-terminally to A(0) or B(0), respectively. Thus A(-2) and B(-2) indicate positions of the amino acids N-terminally to A(-1) and B(-1), respectively, A(-3) and B(-3) indicate positions of the amino acids N-terminally to A(-2) and B(-2), respectively, and so forth.

Herein the terms A(0) or B(0) indicate the positions of the amino acids N-terminally to A1 or B1, respectively. The terms A(-1) or B(-1) indicate the positions of the first amino acids N-terminally to A(0) or B(0), respectively. Thus A(-2) and B(-2) indicate positions of the amino acids N-terminally to A(-1) and B(-1), respectively, A(-3) and B(-3) indicate positions of the amino acids N-terminally to A(-2) and B(-2), respectively, and so forth. The terms A22 or B31 indicate the positions of the amino acids C-terminally to A21 or B30, respectively. The terms A23 or B32 indicate the positions of the first amino acids C-terminally to A22 or B31, respectively. Thus A24 and B33 indicate positions of the amino acids C-terminally to A23 and B32, respectively, and so forth.

The term **"no blunting"** as used herein means that when formulated in one formulation both the rapid acting insulin and the acylated insulin has profile of action which is identical or substantially identical with the profile of action, when administering the rapid acting insulin and the acylated insulin in separate formulations.

Herein, the term **"amino acid residue"** is an amino acid from which, formally, a hydroxy group has been removed from a carboxy group and/or from which, formally, a hydrogen atom has been removed from an amino group.

hGlu is homoglutamic acid.

α-Asp is the L-form of -HNCH(CO-)CH₂COOH.

β-Asp is the L-form of -HNCH(COOH)CH₂CO-.

α-Glu is the L-form of -HNCH(CO-)CH₂CH₂COOH.

γ-Glu is the L-form of -HNCH(COOH)CH₂CH₂CO-.

α-hGlu is the L-form of -HNCH(CO-)CH₂CH₂CH₂COOH.

δ-hGlu is the L-form of -HNCH(COOH)CH₂CH₂CH₂CO-.

β-Ala is -NH-CH₂-CH₂-COOH.

Sar is sarcosine (N-methylglycine).

The expression **"an amino acid residue having a carboxylic acid group in the side chain"** designates amino acid residues like Asp, Glu and hGlu. The amino acids can be in either the L- or D-configuration. If nothing is specified it is understood that the amino acid residue is in the L configuration.

The term **"treatment of a disease"** as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

The term "**bolus insulin", "meal-related insulin"** or "**rapid acting insulin"** as used herein means an insulin peptide which has an immediately onset of action and suited to cover the need for insulin during and after the meal.

The term **"diabetes"** or **"diabetes mellitus"** includes type 1 diabetes, type 2 diabetes, gestational diabetes (during pregnancy) and other states that cause hyperglycaemia. The term is used for a metabolic disorder in which the pancreas produces insufficient amounts of insulin, or in which the cells of the body fail to respond appropriately to insulin thus preventing cells from absorbing glucose. As a result, glucose builds up in the blood.

Type 1 diabetes, also called insulin-dependent diabetes mellitus (IDDM) and juvenile-onset diabetes, is caused by B-cell destruction, usually leading to absolute insulin deficiency.

Type 2 diabetes, also known as non-insulin-dependent diabetes mellitus (NIDDM) and adult-onset diabetes, is associated with predominant insulin resistance and thus relative insulin deficiency and/or a predominantly insulin secretory defect with insulin resistance.

The term **"buffer"** as used herein refers to a chemical compound in a pharmaceutical composition that reduces the tendency of pH of the composition to change over time as would otherwise occur due to chemical reactions. Buffers include chemicals such as sodium phosphate, TRIS, glycyl glycine, sodium acetate and sodium citrate.

The term "**preservative**" as used herein refers to a chemical compound which is added to a pharmaceutical formulation to prevent or delay microbial activity (growth and metabolism). Examples of pharmaceutically acceptable preservatives are phenol, metacresol (m-cresol) and a mixture of phenol and m-cresol.

The term **"isotonicity agent"** as used refers to a chemical compound in a pharmaceutical formulation that serves to modify the osmotic pressure of the pharmaceutical formulation so that the osmotic pressure becomes closer to that of human plasma. Isotonicity agents include Sodium chloride, glycerol, mannitol, propylene glycol etc.

The term "**stabilizer**" as used herein refers to chemicals added to peptide containing pharmaceutical formulations in order to stabilize the peptide, i.e. to increase the shelf life and/or in-use time of such formulations. Examples of stabilizers used in pharmaceutical formulations are L-glycine, L-histidine, arginine, polyethylene glycol, and carboxymethylcellulose. Further phenols, zinc ions and sodium chloride can act as stabilizers.

The term **"surfactant"** as used herein refers to a chemical compound in a pharmaceutical formulation that serves to modify the interface to air and hydrophobic surfaces in a way that displaces or partly displaces insulin, insulin analogues and insulin derivatives from the interfaces. Various conventional surfactants can be employed, such as polyoxyethylene fatty acid esters and alcohols, and polyoxyethylene sorbitol fatty acid esters. An example is polysorbate 20.

### DESCRIPTION OF THE INVENTION

The present invention concerns a process for preparing a pharmaceutical formulation comprising an insulin derivative, wherein the process comprises dissolving an insulin derivative in water, optionally comprising pharmaceutically acceptable excipients, to form a solution of insulin derivative, adjusting the pH of the solution to a pH above 7.2, adding a zinc solution during a period longer than seven minutes while stirring continuously and adjusting the pH to the target pH of the formulation, and wherein the insulin derivative comprises and insulin molecule having a side chain attached to the ε-amino group of a Lys residue present in the B chain of the parent insulin, the side chain being of the general formula:

-W-X-Y-Z

wherein W is:
an α-amino acid residue having a carboxylic acid group in the side chain which residue forms, with one of its carboxylic acid groups, an amide group together with ε-amino group of a Lys residue present in the B chain of the parent insulin;
a chain composed of two, three or four α-amino acid residues linked together via amide carbonyl bonds, which chain - via an amide bond - is linked to an ε-amino group of a Lys residue present in the B chain of the parent insulin, the amino acid residues of W being selected from the group of amino acid residues having a neutral side chain and amino acid residues having a carboxylic acid group in the side chain so that W has at least one amino acid residue which has a carboxylic acid group in the side chain ; or
a covalent bond from X to an ε-amino group of a Lys residue present in the B chain of the parent insulin;
X is:

   -CO-;

   -CH(COOH)CO-;

   -CO-N(CH₂COOH)CH₂CO-;

   -CO-N(CH₂COOH)CH₂CON(CH₂COOH)CH₂CO-;

   -CO-N(CH₂CH₂COOH)CH₂CH₂CO-;

   -CO-N(CH₂CH₂COOH)CH₂CH₂CON(CH₂CH₂COOH)CH₂CH₂CO-;

   -CO-NHCH(COOH)(CH₂)₄NHCO- ;

   -CO-N(CH₂CH₂COOH)CH₂CO-; or

   -CO-N(CH₂COOH)CH₂CH₂CO-.

   that
   when W is an amino acid residue or a chain of amino acid residues, *via* a bond from the underscored carbon forms an amide bond with an amino group in W, or
   when W is a covalent bond, *via* a bond from the underscored carbonyl carbon forms an amide bond with an ε-amino group of a Lys residue present in the B chain of the parent insulin;
Y is:
   -(CH₂)ₘ- where m is an integer in the range of 6 to 32;
   a divalent hydrocarbon chain comprising 1, 2 or 3 -CH=CH- groups and a number of -CH₂- groups sufficient to give a total number of carbon atoms in the chain in the range of 10 to 32; and
Z is:

   -COOH;

   -CO-Asp;

   -CO-Glu;

   -CO-Gly;

   -CO-Sar;

   -CH(COOH)₂;

   -N(CH₂COOH)₂;

   -SO₃H; or

   -PO₃H
and any Zn²⁺ complexes thereof, provided that when W is a covalent bond and X is -CO-, then Z is different from -COOH.

The inventors have surprisingly found that by raising the pH of the solution comprising insulin derivative to a pH value above 7.2, there will be substantially no precipitation of the insulin derivative when the zinc solution is added meaning that no precipitate is formed or if precipitate is formed then it solubilises again at once.
Precipitation of insulin derivative in the solution can be seen by visual inspection of the solution. If the insulin derivative precipitates in the solution, the precipitate renders the solution unclear. When the solution is clear and transparent no precipitation or substantially no precipitation of insulin derivative is present.

In one aspect of the invention the water, wherein the insulin derivative is dissolved, comprises one or more pharmaceutically acceptable excipients when the insulin derivative is dissolved in the water. Various pharmaceutically acceptable excipients such as phenol, m-cresol, glycerol, sodium chloride and optionally TRIS or phosphate buffers can be added to the water to obtain an aqueous solution of excipients and the insulin derivative is dissolved in the aqueous solution.

In one aspect of the invention, one or more pharmaceutically acceptable excipients are added to the aqueous solution of insulin derivative before the pH of the solution is adjusted to the target pH. In one aspect of the invention, the pharmaceutically acceptable excipients are added to the formulation after target pH is adjusted.
In one aspect the pharmaceutically acceptable excipients are selected from the group consisting of phenol, m-cresol, glycerol and sodium chloride.
In one aspect of the invention the target pH is below the pH of the aqueous solution, whereto the zinc solution is added. In one aspect of the invention the pH of the aqueous solution is adjusted to be above 7.4 when the zinc solution is added. In one aspect of the invention the pH of the aqueous solution is adjusted to be above 7.6 when the zinc solution is added. In one aspect of the invention the pH of the aqueous solution is adjusted to be above 7.8 when the zinc solution is added. In one aspect of the invention the pH of the aqueous solution is adjusted to be above 8.0 when the zinc solution is added.
In one aspect of the invention the target pH is in the range of 7.0 to 7.8. In one aspect the target pH is in the range of 7.2 to 7.8. In one aspect the target pH is in the range of 7.4 to 7.6.
In one aspect of the invention the pH of the aqueous solution is adjusted to be above 7.4, the insulin solution is added and the pH is then adjusted to a target pH in the range of 7.0-7.8.
In one aspect the pH of the aqueous solution is adjusted to be above 7.6, the insulin solution is added and the pH is then adjusted to a target pH in the range of 7.0-7.8.
In one aspect the pH of the aqueous solution is adjusted to be above 7.8, the insulin solution is added and the pH is then adjusted to a target pH in the range of 7.0-7.8.
In one aspect the pH of the aqueous solution is adjusted to be above 8.0, the insulin solution is added and the pH is then adjusted to a target pH in the range of 7.0-7.8.
In one aspect the pH of the aqueous solution is adjusted to be above 7.2, the insulin solution is added and the pH is then adjusted to a target pH in the range of 7.2-7.8.
In one aspect the pH of the aqueous solution is adjusted to be above 7.4, the insulin solution is added and the pH is then adjusted to a target pH in the range of 7.2-7.8.
In one aspect the pH of the aqueous solution is adjusted to be above 7.6, the insulin solution is added and the pH is then adjusted to a target pH in the range of 7.2-7.8.
In one aspect the pH of the aqueous solution is adjusted to be above 7.8, the insulin solution is added and the pH is then adjusted to a target pH in the range of 7.2-7.8.
In one aspect the pH of the aqueous solution is adjusted to be above 8.0, the insulin solution is added and the pH is then adjusted to a target pH in the range of 7.2-7.8.

In one aspect the pH of the aqueous solution is adjusted to be above 7.2, the insulin solution is added and the pH is then adjusted to a target pH in the range of 7.4 to 7.6.
In one aspect the pH of the aqueous solution is adjusted to be above 7.4, the insulin solution is added and the pH is then adjusted to a target pH in the range of 7.4 to 7.6.
In one aspect the pH of the aqueous solution is adjusted to be above 7.6, the insulin solution is added and the pH is then adjusted to a target pH in the range of 7.4 to 7.6.
In one aspect the pH of the aqueous solution is adjusted to be above 7.8, the insulin solution is added and the pH is then adjusted to a target pH in the range of 7.4 to 7.6.
In one aspect the pH of the aqueous solution is adjusted to be above 8.0, the insulin solution is added and the pH is then adjusted to a target pH in the range of 7.4 to 7.6.
Various acids and bases can be used for the adjustment of pH in the aqueous solution or to reach the target pH. Examples of suitable acids are hydrochloric acid, acetic acid, sulphuric
acid and phosphoric acid. Examples of suitable bases are sodium hydroxide, TRIS, carbonates and phosphates. In one embodiment TRIS, carbonates and phosphates also act as a buffer.

In one aspect of the invention the zinc solution is added to the aqueous solution during a period longer than seven minutes.
In one aspect of the invention the zinc solution comprises zinc acetate. In one aspect the zinc solution is selected from the group consisting of zinc acetate, zinc chloride, zinc sulphate and zinc gluconate. In one aspect of the invention the zinc solution is zinc acetate.
In one aspect of the invention the proportion of the zinc solution and the soluble insulin derivative is from 4.3 zinc atoms per 6 molecules of insulin derivative to 12 zinc atoms per 6 molecules of insulin derivative. In one aspect of the invention the proportion is between 4.5 and 12 zinc atoms per 6 molecules of insulin derivative.
In one aspect of the invention the proportion is between 4.7 and 12 zinc atoms per 6 molecules of insulin derivative. In one aspect of the invention the proportion is between 4.9 and 12 zinc atoms per 6 molecules of insulin derivative. In one aspect of the invention the proportion is between 5.1 and 12 zinc atoms per 6 molecules of insulin derivative. In one aspect of the invention the proportion is between 5.3 and 12 zinc atoms per 6 molecules of insulin derivative. In one aspect of the invention the proportion is between 5.5 and 12 zinc atoms per 6 molecules of insulin derivative. In one aspect of the invention the proportion is between 5.7 and 12 zinc atoms per 6 molecules of insulin derivative. In one aspect of the invention the proportion is between 5.9 and 11.5 zinc atoms per 6 molecules of insulin derivative. In one aspect of the invention the proportion is between 6.1 and 11.0 zinc atoms per 6 molecules of insulin derivative. In one aspect of the invention the proportion is between 6.3 and 10.5 zinc atoms per 6 molecules of insulin derivative. In one aspect of the invention the proportion is between 6.5 and 10.0 zinc atoms per 6 molecules of insulin derivative.

In one aspect of the invention the insulin derivative is LysB29Nε-hexadecandioyl-γ-Glu desB30 human insulin.

In one aspect of the invention a rapid acting insulin is added to the formulation. The rapid acting insulin can be selected from the group consisting of AspB28 human insulin, LysB3 GluB29 human insulin and/or LysB28 ProB29 human insulin. In one aspect of the invention the rapid acting insulin is AspB28 human insulin (Insulin Aspart).
The invention further concerns a product obtainable by the process for preparing a pharmaceutical formulation comprising an insulin derivative. The product obtainable by the process of the invention can comprise a rapid acting insulin, such as insulin aspart and no blunting occurs.
In one aspect of the invention the use of a product obtainable by the process for preparing a pharmaceutical formulation comprising an insulin derivative for the treatment of diabetes is provided.

In a further aspect of the invention the formulation further comprises a pharmaceutically acceptable preservative which may be selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further aspect of the invention the preservative is present in a concentration from 0.1 mg/ml to 20 mg/ml. In a further aspect of the invention the preservative is present in a concentration from 0.1 mg/ml to 5 mg/ml. In a further aspect of the invention the preservative is present in a concentration from 5 mg/ml to 10 mg/ml. In a further aspect of the invention the preservative is present in a concentration from 10 mg/ml to 20 mg/ml. Each one of these specific preservatives constitutes an alternative aspect of the invention. The use of a preservative in pharmaceutical compositions is well-known to the skilled person. For convenience reference is made to Remington: The Science and Practice of Pharmacy, 19th edition, 1995.

In a further aspect of the invention the formulation further comprises an isotonic agent which may be selected from the group consisting of a salt (e.g. sodium chloride), a sugar or sugar alcohol, an amino acid (e.g. glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one aspect the sugar additive is sucrose. Sugar alcohol is defined as a C4-C8 hydrocarbon having at least one-OH group and includes, for example, mannitol, sorbitol, inositol, galactitol, dulcitol, xylitol, and arabitol. In one aspect the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one aspect, the sugar or sugar alcohol concentration is between about 1 mg/ml and about 150 mg/ml. In a further aspect of the invention the isotonic agent is present in a concentration from 1 mg/ml to 50 mg/ml. In a further aspect of the invention the isotonic agent is present in a concentration from 1 mg/ml to 7 mg/ml. In a further aspect of the invention the isotonic agent is present in a concentration from 8 mg/ml to 24 mg/ml. In a further aspect of the invention the isotonic agent is present in a concentration from 25 mg/ml to 50 mg/ml. Each one of these specific isotonic agents constitutes an alternative aspect of the invention. The use of an isotonic agent in pharmaceutical compositions is well-known to the skilled person. For convenience reference is made to Remington: The Science and Practice of Pharmacy, 19th edition, 1995.

Typical isotonic agents are sodium chloride, mannitol, dimethyl sulfone and glycerol and typical preservatives are phenol, m-cresol, methyl p-hydroxybenzoate and benzyl alcohol.

Examples of suitable buffers are sodium acetate, glycylglycine, HEPES (4-(2-hydroxyethyl)-1-piperazineethanesulfonic acid) and sodium phosphate.

Formulations of this invention can be used in the treatment of states which are sensitive to insulin. Thus, they can be used in the treatment of type 1 diabetes, type 2 diabetes and hyperglycaemia for example as sometimes seen in seriously injured persons and persons who have undergone major surgery. The optimal dose level for any patient will depend on a variety of factors including the efficacy of the specific insulin derivative employed, the age, body weight, physical activity, and diet of the patient, on a possible combination with other drugs, and on the severity of the state to be treated. It is recommended that the daily dosage of the formulation of this invention be determined for each individual patient by those skilled in the art in a similar way as for known insulin formulations.

Where expedient, the insulin derivatives of this invention may be used in mixture with other types of insulin, e.g. insulin analogues with a more rapid onset of action. Examples of such insulin analogues are described e.g. in the European patent applications having the publication Nos. EP 214826 (Novo Nordisk A/S), EP 375437 (Novo Nordisk A/S) and EP 383472 (Eli Lilly & Co.).

In another aspect the present invention provides a product obtainable by the process as defined herein. In yet another aspect the present invention provides the use of the process as defined herein for the manufacture of a medicament for the treatment of diabetes.

The present invention is further illustrated by the following.

### EXAMPLES

### Example 1

### Process for preparing a formulation comprising LysB29Nε-hexadecandioyl-γ-Glu desB30 human insulin, 600 nmol/ml (100U/ml):

0.6 mmol LysB29Nε-hexadecandioyl-γ-Glu desB30 human insulin was dissolved in 300 ml water and mixed with 500 ml of an aqueous solution containing 16 mmol phenol, 16 mmol m-cresol and 213 mmol glycerol. pH was adjusted to 7.40 and 50 ml 0.01 M zinc acetate was added continuously by use of a peristaltic pump while stirring at moderate speed. The addition was done over approximately 30 minutes. After addition of zinc acetate, water for injection was added to 950 ml, pH was adjusted to 7.60 and finally water was added to final volume of 1 litre.

### Example 2

### Process for preparing a formulation comprising LysB29Nε-hexadecandioyl-γ-Glu desB30 human insulin, 1200 nmol/ml (200U/ml):

1.2 mmol LysB29Nε-hexadecandioyl-γ-Glu desB30 human insulin was dissolved in 300 ml water and mixed with 500 ml of an aqueous solution containing 16 mmol phenol, 16 mmol *m-*cresol and 213 mmol glycerol. pH was adjusted to 7.50 and 110 ml 0.01 M zinc acetate was added continuously by use of a peristaltic pump while stirring at moderate speed. The addition was done over approximately 40 minutes. After addition of zinc acetate, water for injection was added to 950 ml, pH was adjusted to 7.60 and finally water was added to final volume of 1 litre.

### Example 3

### Process for preparing a formulation comprising LysB29Nε-hexadecandioyl-γ-Glu desB30 human insulin and insulin aspart 600 nmol/ml (U100/ml):

**LysB29Nε-hexadecandioyl-γ-Glu desB30 human insulin solution:** 0.42 mmol LysB29Nε-hexadecandioyl-γ-Glu desB30 human insulin was dissolved in 210 ml water and mixed with 350 ml of an aqueous solution containing 11.2 mmol phenol, 11.2 mmol *m*-cresol, 7 mmol NaCl and 144 mmol glycerol. pH was adjusted to 7.40 and 32.9 ml 0.01 M zinc acetate was added continuously by use of a peristaltic pump while stirring at moderate speed. The addition was done over approximately 30 minutes. After addition of zinc acetate, water for injection was added to 630 ml and pH was adjusted to 7.40.

**Insulin aspart solution:** 0.18 mmol insulin aspart was suspended in 15 ml water and mixed with a solution containing 9 ml 0.01 M zinc acetate and 4.8 ml 0.2 N hydrochloric acid to obtain a clear solution. The volume was adjusted to 35 ml by adding water. 180 ml of a solution containing 4.8 mmol phenol, 4.8 mmol m-cresol, 3 mmol NaCl and 62 mmol glycerol was then added. Finally pH was adjusted to 7.40 and the volume was adjusted to 270 ml by adding water.

**Mixing of LysB29Nε-hexadecandioyl-γ-Glu desB30 human insulin solution and insulin aspart solution:** 630 ml of LysB29Nε-hexadecandioyl-γ-Glu desB30 human insulin solution and 270 ml of insulin aspart solution were mixed. pH was adjusted to 7.40 and finally the volume was adjusted to 1 litre by adding water

### Example 4

### Process for preparing a formulation comprising LysB29Nε-hexadecandioyl-γ-Glu desB30 human insulin and insulin aspart, 1200 nmol/ml (200 U/ml):

**LysB29Nε-hexadecandioyl-γ-Glu desB30 human insulin solution:** 0.84 mmol LysB29Nε-hexadecandioyl-γ-Glu desB30 human insulin was dissolved in 210 ml water and mixed with 350 ml of an aqueous solution containing 11.2 mmol phenol, 11.2 mmol *m*-cresol, 7 mmol NaCl and 144 mmol glycerol. pH was adjusted to 7.40 and 60.1 ml 0.01 M zinc acetate was added continuously by use of a peristaltic pump while stirring at moderate speed. The addition was done over approximately 30 minutes. After addition of zinc acetate, water for injection was added to 630 ml and pH was adjusted to 7.40.

**Insulin aspart solution:** 0.36 mmol insulin aspart was suspended in 15 ml water and mixed with a solution containing 18 ml 0.01 M zinc acetate and 4.8 ml 0.2 N hydrochloric acid to obtain a clear solution. The volume was adjusted to 35 ml by adding water. 180 ml of a solution containing 4.8 mmol phenol, 4.8 mmol m-cresol, 3 mmol NaCl and 62 mmol glycerol was then added. Finally pH was adjusted to 7.40 and the volume was adjusted to 270 ml by adding water.

**Mixing of LysB29Nε-hexadecandioyl-γ-Glu desB30 human insulin solution and insulin aspart solution:** 630 ml of LysB29Nε-hexadecandioyl-γ-Glu desB30 human insulin solution and 270 ml of insulin aspart solution were mixed. pH was adjusted to 7.40 and finally the volume was adjusted to 1 litre by adding water.

## Claims

1. A process for preparing a pharmaceutical formulation comprising an insulin derivative, wherein the process comprises dissolving an insulin derivative in water, adjusting the pH of the solution to a pH above 7.2, adding a zinc solution during a period longer than seven minutes while stirring continuously and adjusting the pH to the target pH of the formulation, and wherein the insulin derivative comprises an insulin molecule having a side chain attached to the ε-amino group of a Lys residue present in the B chain of human insulin or an analogue thereof, the side chain being of the general formula:
-W-X-Y-Z
wherein W is:
• an α-amino acid residue having a carboxylic acid group in the side chain which residue forms, with one of its carboxylic acid groups, an amide group together with ε-amino group of a Lys residue present in the B chain of the parent insulin;
• a chain composed of two, three or four α-amino acid residues linked together via amide carbonyl bonds, which chain - via an amide bond - is linked to an ε-amino group of a Lys residue present in the B chain of the parent insulin, the amino acid residues of W being selected from the group of amino acid residues having a neutral side chain and amino acid residues having a carboxylic acid group in the side chain so that W has at least one amino acid residue which has a carboxylic acid group in the side chain ; or
• a covalent bond from X to an ε-amino group of a Lys residue present in the B chain of the parent insulin;
X is:
• -CO-;
• -CH(COOH)CO-;
• -CO-N(CH₂COOH)CH₂CO-;
• -CO-N(CH₂COOH)CH₂CON(CH₂COOH)CH₂CO-;
• -CO-N(CH₂CH₂COOH)CH₂CH₂CO-;
• -CO-N(CH₂CH₂COOH)CH₂CH₂CON(CH₂CH₂COOH)CH₂CH₂CO-;
• -CO-NHCH(COOH)(CH₂)₄NHCO- ;
• -CO-N(CH₂CH₂COOH)CH₂CO-; or
• -CO-N(CH₂COOH)CH₂CH₂CO-.
that
a) when W is an amino acid residue or a chain of amino acid residues, *via* a bond from the underscored carbon forms an amide bond with an amino group in W, or
b) when W is a covalent bond, *via* a bond from the underscored carbonyl carbon forms an amide bond with an ε-amino group of a Lys residue present in the B chain of the parent insulin;
Y is:
• -(CH₂)ₘ- where m is an integer in the range of 6 to 32;
• a divalent hydrocarbon chain comprising 1, 2 or 3 -CH=CH- groups and a number of -CH₂- groups sufficient to give a total number of carbon atoms in the chain in the range of 10 to 32; and
Z is:
• -COOH;
• -CO-Asp;
• -CO-Glu;
• -CO-Gly;
• -CO-Sar;
• -CH(COOH)₂;
• -N(CH₂COOH)₂;
• -SO₃H; or
• -PO₃H.

2. A process according to claim 1, wherein the water comprises one or more pharmaceutically acceptable excipients.

3. A process according to claim 1, wherein one or more pharmaceutically acceptable excipients is added to the formulation after target pH is adjusted.

4. A process according to claims 1-3, wherein the pharmaceutically acceptable excipients are selected from the group consisting of phenol, m-cresol, glycerol and sodium chloride.

5. A process according to claims 1-4, wherein the target pH is below the pH of the water.

6. A process according to claims 1-5, wherein the proportion of the zinc solution to the soluble insulin derivative is from 4.3 zinc atoms per 6 molecules of insulin derivative to 12 zinc atoms per 6 molecules of insulin derivative.

7. A process according to claims 1-6, wherein the target pH is in the range of 7.0 to 7.8.

8. A process according to claims 1-7, wherein the zinc solution comprises zinc acetate.

9. A process according to claim 8, wherein the insulin derivative is LysB29Nε-hexadecandioyl-γ-Glu desB30 human insulin.

10. A process according to claims 1-9, wherein a rapid acting insulin is added to the formulation.

11. A process according to claims 1-10, wherein the rapid acting insulin is selected from the group consisting of AspB28 human insulin, LysB3 GluB29 human insulin and/or LysB28 ProB29 human insulin.

12. A product obtainable by the process of claims 1-11.

13. Use of the product of claim 12 for the manufacture of a medicament for the treatment of diabetes.

## Patentansprüche

1. Verfahren zur Herstellung einer ein Insulinderivat umfassenden pharmazeutischen Formulierung, wobei das Verfahren Lösen eines Insulinderivats in Wasser, Einstellen des pH-Werts der Lösung auf einen pH-Wert oberhalb 7,2, Zugeben einer Zinklösung unter ständigem Rühren über einen Zeitraum von mehr als sieben Minuten und Einstellen des pH-Werts auf den Ziel-pH-Wert der Formulierung umfasst und wobei das Insulinderivat ein Insulinmolekül mit einer an die ε-Aminogruppe eines in der B-Kette von Humaninsulin oder einem Analog davon vorhandenen Lys-Rests gebundenen Seitenkette umfasst, bei der es sich um eine Seitenkette der allgemeinen Formel:
-W-X-Y-Z
handelt, wobei W für:
• einen α-Aminosäurerest mit einer Carbonsäuregruppe in der Seitenkette steht, wobei der Rest über eine seiner Carbonsäuregruppen zusammen mit der ε-Aminogruppe eines in der B-Kette des Ausgangsinsulins vorhandenen Lys-Rests eine Amidgruppe bildet;
• eine aus zwei, drei oder vier miteinander über Amid-Carbonyl-Bindungen verknüpften α-Aminosäureresten zusammengesetzte Kette steht, die - über eine Amidbindung - mit einer ε-Aminogruppe eines in der B-Kette des Ausgangsinsulins vorhandenen Lys-Rests verknüpft ist, wobei die Aminosäurereste von W aus der Gruppe von Aminosäureresten mit einer neutralen Seitenkette und Aminosäureresten mit einer Carbonsäuregruppe in der Seitenkette ausgewählt sind, so dass W wenigstens einen Aminosäurerest besitzt, der eine Carbonsäuregruppe in der Seitenkette aufweist; oder
• eine kovalente Bindung von X zu einer ε-Aminogruppe eines in der B-Kette des Ausgangsinsulins vorhandenen Lys-Rests steht;
X für:
• -CO-;
• -CH(COOH)CO-;
• -CO-N(CH₂COOH)CH₂CO-;
• -CO-N(CH₂COOH)CH₂CON(CH₂COOH)CH₂CO-;
• -CO-N(CH₂CH₂COOH)CH₂CH₂CO-;
• -CO-N(CH₂CH₂COOH)CH₂CH₂CON(CH₂CH₂COOH)CH₂CH₂CO-;
• -CO-NHCH(COOH)(CH₂)₄NHCO-;
• -CO-N(CH₂CH₂COOH)CH₂CO-; oder
• -CO-N(CH₂COOH)CH₂CH₂CO-
steht, das,
a) wenn W für einen Aminosäurerest oder eine Kette von Aminosäureresten steht, über eine Bindung von dem unterstrichenen Kohlenstoff eine Amidbindung mit einer Aminogruppe in W bildet oder,
b) wenn W für eine kovalente Bindung steht, über eine Bindung von dem unterstrichenen Carbonylkohlenstoff eine Amidbindung mit einer ε-Aminogruppe eines in der B-Kette des Ausgangsinsulins vorhandenen Lys-Rests bildet;
Y für:
• -(CH₂)ₘ- steht, worin m eine ganze Zahl im Bereich von 6 bis 32 ist;
• eine divalente Kohlenwasserstoffkette steht, die 1, 2 oder 3 -CH=CH--Gruppen und eine hinreichende Anzahl -CH₂--Gruppen umfasst, so dass sich eine Gesamtzahl von Kohlenstoffatomen in der Kette im Bereich von 10 bis 32 ergibt; und
Z für:
• -COOH;
• -CO-Asp;
• -CO-Glu;
• -CO-Gly;
• -CO-Sar;
• -CH(COOH)₂;
• -N(CH₂COOH)₂;
• -SO₃H; oder
• -PO₃H steht.

2. Verfahren nach Anspruch 1, wobei das Wasser einen oder mehrere pharmazeutisch unbedenkliche Hilfsstoffe umfasst.

3. Verfahren nach Anspruch 1, wobei ein oder mehrere pharmazeutisch unbedenkliche Hilfsstoffe nach Einstellen des Ziel-pH-Werts zur Formulierung gegeben werden.

4. Verfahren nach Anspruch 1-3, wobei die pharmazeutisch unbedenklichen Hilfsstoffe aus der aus Phenol, m-Cresol, Glycerin und Natriumchlorid bestehenden Gruppe ausgewählt sind.

5. Verfahren nach Anspruch 1-4, wobei der Ziel-pH-Wert unterhalb des pH-Werts des Wassers liegt.

6. Verfahren nach Anspruch 1-5, wobei das Verhältnis der Zinklösung zum löslichen Insulinderivat 4,3 Zinkatome pro 6 Moleküle Insulinderivat bis 12 Zinkatome pro 6 Moleküle Insulinderivat beträgt.

7. Verfahren nach Anspruch 1-6, wobei der Ziel-pH-Wert im Bereich von 7,0 bis 7,8 liegt.

8. Verfahren nach Anspruch 1-7, wobei die Zinklösung Zinkacetat umfasst.

9. Verfahren nach Anspruch 8, wobei es sich bei dem Insulinderivat um LysB29Nε-hexadecandioyl-γ-GludesB30-Humaninsulin handelt.

10. Verfahren nach Anspruch 1-9, wobei ein schnell wirkendes Insulin zur Formulierung gegeben wird.

11. Verfahren nach Anspruch 1-10, wobei das schnell wirkende Insulin aus der aus AspB28-Humaninsulin, LysB3-GluB29-Humaninsulin und/oder LysB28-ProB29-Humaninsulin bestehenden Gruppe ausgewählt ist.

12. Produkt, erhältlich mit dem Verfahren nach Anspruch 1-11.

13. Verwendung des Produkts nach Anspruch 12 zur Herstellung eines Arzneimittels zur Behandlung von Diabetes.

## Revendications

1. Procédé de préparation d'une formulation pharmaceutique comprenant un dérivé d'insuline, le procédé comprenant la dissolution d'un dérivé d'insuline dans de l'eau, l'ajustement du pH de la solution à un pH au-dessus de 7,2, l'ajout d'une solution de zinc pendant une durée plus longue que sept minutes sous agitation continue et en ajustant le pH au pH cible de la formulation, et dans lequel le dérivé d'insuline comprend une molécule d'insuline ayant une chaîne latérale liée au groupe ε-amino d'un résidu Lys présent dans la chaîne B de l'insuline humaine ou un analogue de celle-ci, la chaîne latérale étant de formule générale :
-W-X-Y-Z
dans laquelle W est :
• un résidu d'acide α-aminé ayant un groupe acide carboxylique dans la chaîne latérale, ledit résidu formant, avec un de ses groupes acide carboxylique, un groupe amide conjointement avec le groupe ε-amino d'un résidu Lys présent dans la chaîne B de l'insuline parente ;
• une chaîne composée de deux, trois ou quatre résidus d'acide α-aminé liés conjointement par l'intermédiaire de liaisons carbonylamide, ladite chaîne, par l'intermédiaire d'une liaison amide, étant liée à un groupe ε-amino d'un résidu Lys présent dans la chaîne B de l'insuline parente, les résidus d'acide aminé de W étant choisis dans le groupe de résidus d'acide aminé ayant une chaîne latérale neutre et de résidus d'acide aminé ayant un groupe acide carboxylique dans la chaîne latérale de sorte que W ait au moins un résidu d'acide aminé qui a un groupe acide carboxylique dans la chaîne latérale ; ou
• une liaison covalente de X à un groupe ε-amino d'un résidu Lys présent dans la chaîne B de l'insuline parente ;
X est :
• -CO- ;
• -CH(COOH)CO- ;
• -CO-N(CH₂COOH)CH₂CO- ;
• -CO-N(CH₂COOH)CH₂CON(CH₂COOH)CH₂CO- ;
• -CO-N(CH₂CH₂COOH)CH₂CH₂CO- ;
• -CO-N(CH₂CH₂COOH)CH₂CH₂CON(CH₂CH₂COOH)CH₂CH₂CO- ;
• -CO-NHCH(COOH)(CH₂)₄NHCO- ;
• -CO-N(CH₂CH₂COOH)CH₂CO- ; ou
• -CO-N(CH₂COOH)CH₂CH₂CO-,
qui
a) lorsque W est un résidu d'acide aminé ou une chaîne de résidus d'acide aminé, par l'intermédiaire d'une liaison depuis le carbone souligné forme une liaison amide avec un groupe amino dans W, ou
b) lorsque W est une liaison covalente, par l'intermédiaire d'une liaison depuis le carbone de carbonyle souligné forme une liaison amide avec un groupe ε-amino d'un résidu Lys présent dans la chaîne B de l'insuline parente ;
Y est :
• -(CH₂)ₘ- où m est un entier dans la plage de 6 à 32 ;
• une chaîne hydrocarbonée divalente comprenant 1, 2 ou 3 groupes -CH=CH- et un nombre de groupes -CH₂-suffisants pour obtenir un nombre total d'atomes de carbone dans la chaîne dans la plage de 10 à 32 ; et
Z est :
• -COOH ;
• -CO-Asp ;
• -CO-Glu ;
• -CO-Gly ;
• -CO-Sar ;
• -CH(COOH)₂ ;
• -N(CH₂COOH)₂ ;
• -SO₃H ; ou
• -PO₃H.

2. Procédé selon la revendication 1, dans lequel l'eau comprend un ou plusieurs excipients pharmaceutiquement acceptables.

3. Procédé selon la revendication 1, dans lequel un ou plusieurs excipients pharmaceutiquement acceptables sont ajoutés à la formulation une fois que le pH cible est ajusté.

4. Procédé selon les revendications 1 à 3, dans lequel les excipients pharmaceutiquement acceptables sont choisis dans le groupe constitué des phénol, m-crésol, glycérol et chlorure de sodium.

5. Procédé selon les revendications 1 à 4, dans lequel le pH cible est inférieur au pH de l'eau.

6. Procédé selon les revendications 1 à 5, dans lequel la proportion de la solution de zinc par rapport au dérivé d'insuline soluble est de 4,3 atomes de zinc par 6 molécules de dérivé d'insuline à 12 atomes de zinc par 6 molécules de dérivé d'insuline.

7. Procédé selon les revendications 1 à 6, dans lequel le pH cible est dans la plage de 7,0 à 7,8.

8. Procédé selon les revendications 1 à 7, dans lequel la solution de zinc comprend de l'acétate de zinc.

9. Procédé selon la revendication 8, dans lequel le dérivé d'insuline est l'insuline humaine LysB29Nε-hexadécanedioyl-γ-Glu desB30.

10. Procédé selon les revendications 1 à 9, dans lequel une insuline à action rapide est ajoutée à la formulation.

11. Procédé selon les revendications 1 à 10, dans lequel l'insuline à action rapide est choisie dans le groupe constitué de l'insuline humaine AspB28, l'insuline humaine LysB3 GluB29 et/ou l'insuline humaine LysB28 ProB29.

12. Produit pouvant être obtenu par le procédé des revendications 1 à 11.

13. Utilisation du produit de la revendication 12 pour la fabrication d'un médicament pour le traitement du diabète.
